# EUROPEAN PATENT APPLICATION

(11) **EP 4 684 752 A1**
(43) Date of publication of application: **28.01.2026**
(21) Application number: 24927632.0
(22) Date of filing: 10.04.2024
(51) Int. Cl.: A61B 34/37

(54) **OPERATION SUPPORT DEVICE**

(71) Applicant: RIVERFIELD Inc., Tokyo 107-0052 (JP)
(72) Inventor: MIYASAKA, Muneaki, Tokyo 107-0052 (JP); TADANO, Kotaro, Tokyo 107-0052 (JP)
(74) Representative: Adares PartGmbB
(86) International application number: PCT/JP2024/014511
(87) International publication number: WO 2025/215759

(57) **Abstract**

A manipulation assisting device according to the present invention includes a manipulation unit to be manipulated by a manipulator, an arm unit including a first movable part turnable in a direction around a first turning axis and making the position and attitude of the manipulation unit adjustable, and a placement part provided with a placement surface on which part of a forearm used for the manipulation of the manipulation unit by the manipulator is to be placed. The first turning axis is located at a position at which the distance between the first turning axis and a wrist of a forearm of the manipulator is shorter than the distance between the manipulation unit and the wrist when a manipulation of the manipulation unit is performed by a turn in which the wrist of the forearm is the center of turn with the forearm placed on the placement surface.

## Description

### Technical Field

The present invention relates to a technique for a manipulation assisting device that assists manipulation in surgery or the like.

### Background Art

There is an assistance device that allows a manipulator to perform finer manipulations. For example, in the medical field, there is a type of surgery known as microsurgery, which requires fine manipulations. In the microsurgery, a device with the function of supporting manipulations may be used when a surgeon as the manipulator performs surgery on a patient (e.g., Patent Literature 1).

### Citation List

### Patent Literature

Patent Literature 8: WO 2023/127025

### Summary of Invention

### Technical Problem

The device according to Patent Literature 1 is a surgery assisting system including a master device and a slave device. In the surgery assisting system, the master device is manipulated to operate the slave device remotely placed, thus implementing surgery on a patient.

Unfortunately, the device manipulated by the surgeon tends to increase in size, causing a problem of a limited space for installation and a problem in that nothing can be placed around the device.

The present invention is made in view of the above circumstances, and an object of the present invention is to reduce the size of the device to improve the usability of the device.

### Solution to Problem

A manipulation assisting device according to the present invention is a manipulation assisting device that includes a manipulation unit to be manipulated by a manipulator, an arm unit including a first movable part turnable in a direction around a first turning axis and making the position and attitude of the manipulation unit adjustable, and a placement part provided with a placement surface on which part of a forearm used for the manipulation of the manipulation unit by the manipulator is to be placed. The first turning axis is located at a position at which the distance between the first turning axis and a wrist of a forearm of the manipulator is shorter than the distance between the manipulation unit and the wrist when manipulation of the manipulation unit is performed by a turn in which the wrist of the forearm is the center of turn with the forearm placed on the placement surface.

Assuming that a "wrist turning manipulation" denotes manipulation of the manipulation unit by a turn in which the wrist is the center of turn, the above-described configuration makes a large part of the area within which a hand moves in the wrist turning manipulation and a large part of an area within which the arm unit moves in the wrist turning manipulation overlap with each other.

### Advantageous Effects of Invention

According to the present invention, it is possible to reduce the size of the device to improve the usability of the device.

### Brief Description of Drawings

FIG. 1 is a block diagram illustrating a configuration example of a surgery assisting system in an embodiment of the present invention.
FIG. 2 is a block diagram illustrating a configuration example of a master device.
FIG. 3 is a block diagram illustrating a configuration example of a master device including parts for a right hand and parts for a left hand.
FIG. 4 is a block diagram illustrating a specific configuration example of the master device.
FIG. 5 is a perspective view illustrating a configuration example of the master device.
FIG. 6 is a perspective view illustrating how the master device is manipulated.
FIG. 7 is a perspective view in which some parts are not illustrated for the description of a second movable part in the master device.
FIG. 8 is a perspective view illustrating a first movable part and its surroundings in an extractive manner.
FIG. 9 is a perspective view in which some parts in the vicinity of the first movable part are not illustrated for the description of a first turning axis.
FIG. 10 is a perspective view illustrating a state in which the first movable part is turned.
FIG. 11 is a schematic diagram for describing the second movable part.
FIG. 12 is a schematic diagram for describing a third movable part.
FIG. 13 is a schematic diagram illustrating a state in which the third movable part is turned.
FIG. 14 is a schematic diagram for describing a fourth movable part, a fifth movable part, and a sixth movable part.
FIG. 15 is a schematic perspective view for describing a seventh movable part.
FIG. 16 is a schematic diagram illustrating a state in which the seventh movable part is closed with respect to a grasping part.
FIG. 17 is a schematic diagram illustrating a state in which the seventh movable part is opened with respect to the grasping part.
FIG. 18 is a diagram for describing the first turning axis.
FIG. 19 is a diagram for describing the first turning axis.
FIG. 20 is a diagram illustrating states before and after a foundation turns with respect to an arm rest.
FIG. 21 is a diagram for describing that most of the region where a part distal to a wrist of a surgeon can be present and most of the region where a base part of the foundation can be present overlap each other.
FIG. 22 is a diagram for describing the positional relationship among the center of turn of the wrist, a manipulation unit, and the first turning axis in terms of another indicator.
FIG. 23 is a diagram illustrating a modification example of the configuration of the arm rest.
FIG. 24 is a block diagram illustrating a configuration example of a simulation system.

### Description of Embodiments

### <1. Configuration of Manipulation Assisting System>

A configuration of a manipulation assisting system S according to an embodiment will be described below with reference to the attached drawings.

An embodiment of the manipulation assisting system S is a surgery assisting system SA.

Note that each configuration illustrated in the drawings referred to is merely an example for realizing the present invention. Therefore, various changes can be made depending on, for example, the design without departing from the technical idea of the present invention. Further, in order to avoid duplicate explanation, components that have been described once may be given the same reference numerals and repeated explanations may be omitted.

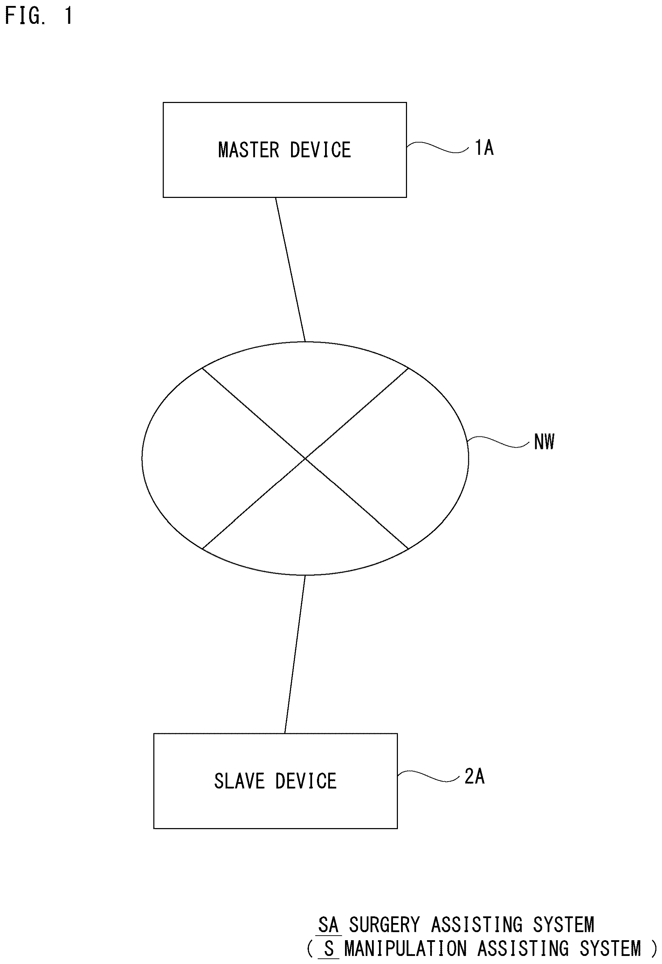

As illustrated in FIG. 1, the surgery assisting system SA includes a master device 1A and a slave device 2A. The master device 1A and the slave device 2A are connected together over a wired or wireless communication network NW, such as the Internet. This enables the slave device 2A to receive information on an input manipulation to the master device 1A and enables the master device 1A to receive information detected by the slave device 2A.

In the surgery assisting system SA, parts included in the slave device 2A are driven in accordance with manipulation of the master device 1A by a surgeon, and thus the surgery on a patient progresses.

The master device 1A is a device used by a surgeon (doctor) being a manipulator who performs surgery. The slave device 2A is a device installed in an operating room where a patient to be operated on is lying down and performs an actual surgery on the patient.

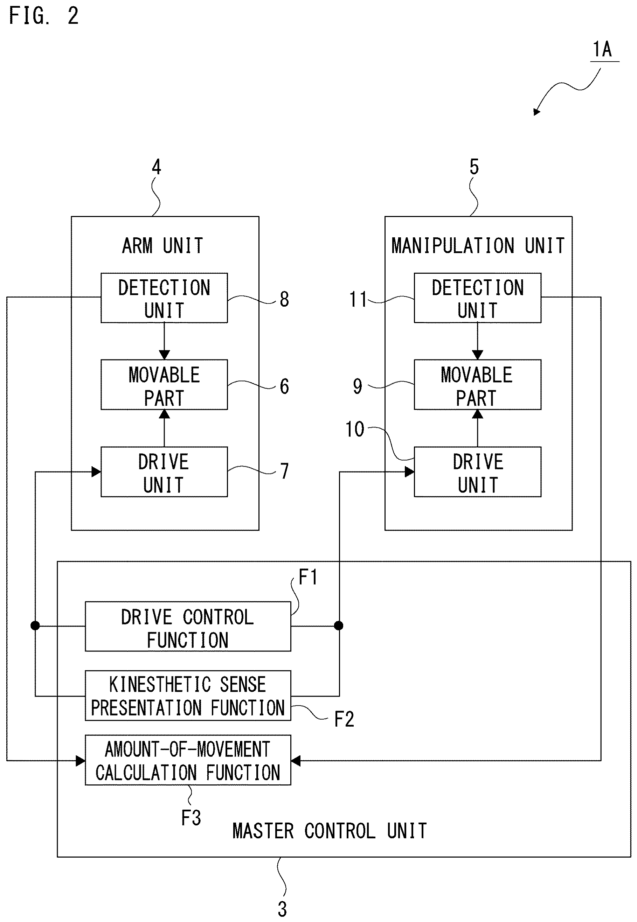

As illustrated in FIG. 2, the master device 1A includes an arm unit 4 and a manipulation unit 5 that is manipulated by the surgeon. The arm unit 4 is provided as a mechanism that changes the position and attitude of the manipulation unit 5 in response to the manipulation by the surgeon.

The slave device 2A includes a holding mechanism that holds a surgical instrument such as forceps or a scalpel, and an adjusting mechanism that changes the position and attitude of the holding mechanism. Although the configuration of the slave device 2A is not illustrated, the adjusting mechanism of the slave device 2A adjusts the parts such that the position and attitude of the surgical instrument held by the holding mechanism match the position and attitude of the manipulation unit 5 of the master device 1A.

This enables the surgeon to handle the surgical instrument of the slave device 2A, which is located at a remote location, at will by manipulating the manipulation unit 5 of the master device 1A.

The master device 1A and the slave device 2A are installed separate from each other. The master device 1A and the slave device 2A may be installed in separate rooms or may be installed in the same room. That is, the master device 1A and the slave device 2A may be installed in the same room and connected together in a wired manner.

In the embodiment described below, the illustration of the specific configuration of the slave device 2A will be omitted.

The configuration of the master device 1A will be described with reference to FIG. 2. Note that FIG. 2 illustrates only parts relating to control out of the parts included in the master device 1A.

The master device 1A includes a master control unit 3, and the above-described arm unit 4 and manipulation unit 5 that are controlled by the master control unit 3.

The master control unit 3 includes a central processing unit (CPU), a read only memory (ROM), a random access memory (RAM), and the like. The master control unit 3 implements predetermined functions with an arithmetic processing unit, such as the CPU, executing a program stored in the ROM or a program loaded into the RAM.

The arm unit 4 includes a plurality of movable parts 6. The arm unit 4 further includes a drive unit 7 that drives the movable parts 6 and a detection unit 8 that detects the amounts of movements of the movable parts 6.

As with the arm unit 4, the manipulation unit 5 includes a movable part 9, a drive unit 10, and a detection unit 11.

The movable parts 6 of the arm unit 4 and the movable part 9 of the manipulation unit 5 are configured to be capable of making a movement such as a turn. Some of the movable parts 6 and movable part 9 may each be configured to be movable by means of a parallel linkage.

The drive unit 7 of the arm unit 4 and the drive unit 10 of the manipulation unit 5 are each configured as an actuator such as a motor. The drive unit 7 and the drive unit 10 are driven to prevent the movable parts 6 and the movable part 9 from naturally turning due to gravity. That is, the drive unit 7 and the drive unit 10 are driven to maintain the position and attitude of the manipulation unit 5 that are changed by the surgeon.

This makes it easy for the surgeon to keep the state in which the manipulation unit 5 is held at an intended position and in an intended attitude.

The drive unit 7 and the drive unit 10 are also driven to cause the manipulation unit 5 of the master device 1A to reproduce a tactile sensation and a pressure such as a resistance force that are received by the surgical instrument of the slave device 2A. This function is the function of feeding a tactile sensation back to the surgeon.

The master control unit 3 supplies the drive unit 7 and the drive unit 10 with control signals to drive the drive unit 7 and the drive unit 10, thus implementing the movements of the movable parts 6 and the movable part 9. That is, the master control unit 3 functions as a drive control function F1.

The master control unit 3 also implements a kinesthetic sense presentation function F2 of feeding back a tactile sensation. Note that the kinesthetic sense presentation function F2 may be implemented as an aspect of the function of the drive control function F1.

The master control unit 3 receives detection signals of detected amounts of movements of the movable parts 6 and the movable part 9 from the detection unit 8 and the detection unit 11, respectively. On the basis of the detection signals received from the detection unit 8 and the detection unit 11, the master control unit 3 calculates the amounts of movements of the movable parts 6 and the movable part 9 and converts the amounts of movements into the amounts of movements of corresponding movable parts of the slave device 2A. That is, the master control unit 3 functions as an amount-of-movement calculation function F3.

Note that the process of the conversion may be executed by the slave device 2A.

In the slave device 2A, its movable parts are moved in accordance with the converted amounts of movements.

Note that it can be assumed that the surgeon manipulates the master device 1A with both hands of the surgeon. Specifically, the master device 1A may be provided with an arm unit 4 and a manipulation unit 5 for a right hand and with an arm unit 4 and a manipulation unit 5 for a left hand.

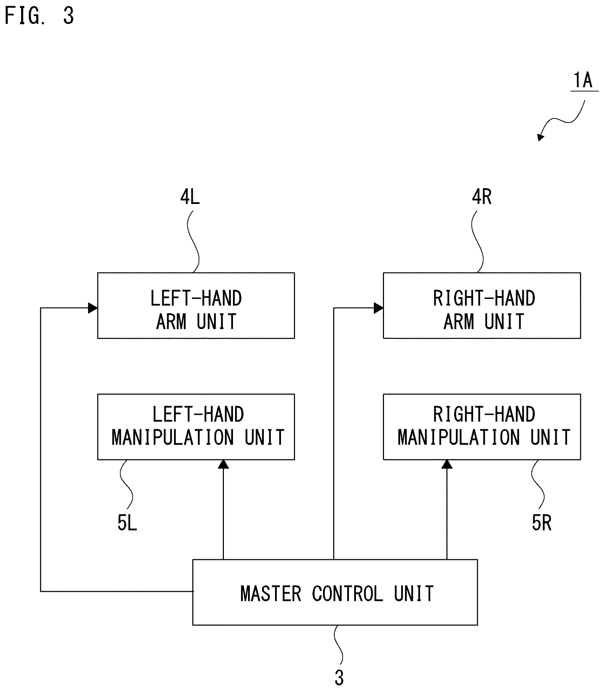

For example, as illustrated in FIG. 3, the master device 1A may include the master control unit 3, a right-hand arm unit 4R and a right-hand manipulation unit 5R, and a left-hand arm unit 4L and a left-hand manipulation unit 5L.

Note that the master device 1A is not limited to this and may be provided with an arm unit 4 and a manipulation unit 5 for a left foot or a right foot. Furthermore, in the case where there are a plurality of surgeons, there may be an arm unit 4 and a manipulation unit 5 provided for a surgeon A and an arm unit 4 and a manipulation unit 5 provided for a surgeon B.

That is, various configurations of the arm unit 4 and the manipulation unit 5 are conceivable.

The configuration of the right-hand arm unit 4R and the right-hand manipulation unit 5R can be the same as the configuration of the left-hand arm unit 4L and the left-hand manipulation unit 5L. Specifically, totally identical configurations are arranged separate from each other, and the arm unit 4 and the manipulation unit 5 located on the left of the surgeon are regarded as those for left hand, and the arm unit 4 and the manipulation unit 5 located on the right of the surgeon are regarded as those for right hand. This enables the arm units 4 to be produced as identical parts and enables the manipulation units 5 to be produced as identical parts, and it is thus possible to reduce costs or use parts commonly.

The configuration of the right-hand arm unit 4R and the right-hand manipulation unit 5R and the configuration of the left-hand arm unit 4L and the left-hand manipulation unit 5L may be symmetrical.

In the following description, the arm unit 4 and the manipulation unit 5 are assumed to be the right-hand arm unit 4R and the right-hand manipulation unit 5R unless otherwise stated. The description of the configuration of the left-hand arm unit 4L and the left-hand manipulation unit 5L will be omitted because their configuration is the same as that of the right-hand arm unit 4R and the right-hand manipulation unit 5R.

### <2. Configuration Example of Master Device>

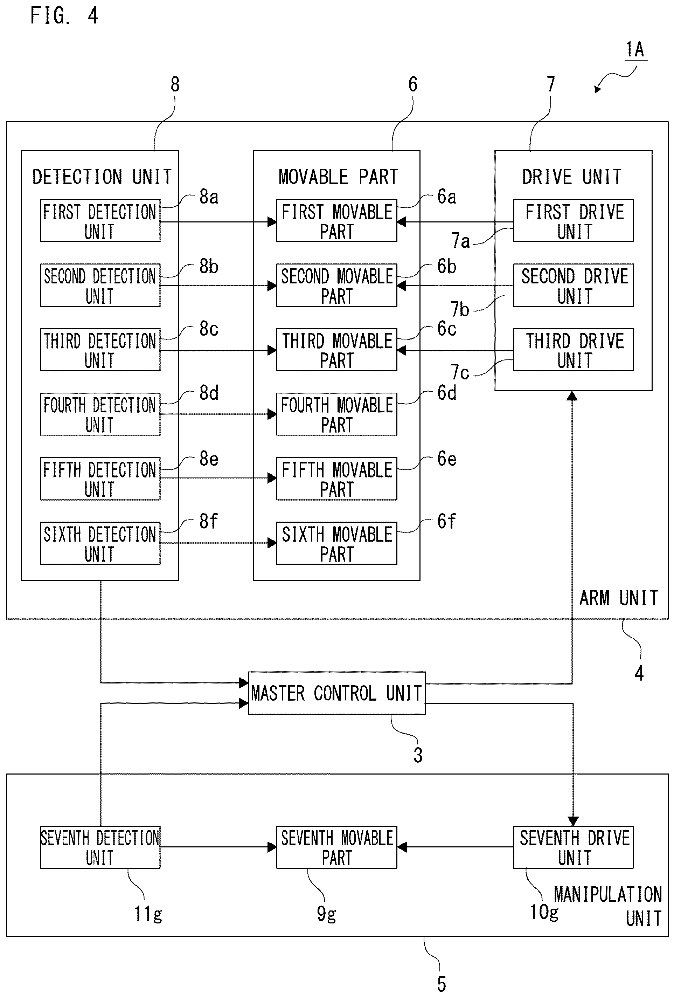

The arm unit 4 included in the master device 1A includes the plurality of movable parts 6. Specifically, as illustrated in FIG. 4, the arm unit 4 is provided with, as the movable parts 6 with six degrees of freedom (6DoF), a first movable part 6a, a second movable part 6b, a third movable part 6c, a fourth movable part 6d, a fifth movable part 6e, and a sixth movable part 6f.

The drive unit 7 included in the master device 1A may be provided for each of the movable parts 6. Note that no drive unit 7 may be provided for some of the movable parts 6. In the example illustrated in FIG. 4, the master device 1A is provided with only a first drive unit 7a corresponding to the first movable part 6a, a second drive unit 7b corresponding to the second movable part 6b, and a third drive unit 7c corresponding to the third movable part 6c.

The detection unit 8 included in the master device 1A may be provided for each of the movable parts 6. Specifically, the master device 1A is provided with a first detection unit 8a corresponding to the first movable part 6a, a second detection unit 8b corresponding to the second movable part 6b, a third detection unit 8c corresponding to the third movable part 6c, a fourth detection unit 8d corresponding to the fourth movable part 6d, a fifth detection unit 8e corresponding to the fifth movable part 6e, and a sixth detection unit 8f corresponding to the sixth movable part 6f.

The manipulation unit 5 included in the master device 1A includes one movable part 9. The movable part 9 included in the manipulation unit 5 is a movable part 9 for giving the master device 1A the seventh degree of freedom and will hereinafter be denoted as a seventh movable part 9g.

Likewise, the detection unit 11 included in the manipulation unit 5 will be denoted as a seventh detection unit 11g corresponding to the seventh movable part 9g.

Note that although the manipulation unit 5 includes, as a drive unit 10, a seventh drive unit 10g corresponding to the seventh movable part 9g, the seventh drive unit 10g is not essential. The following will describe an example in which the manipulation unit 5 is provided with the seventh drive unit 10g.

The master control unit 3 receives detection signals of the detected amounts of movements of the first movable part 6a, the second movable part 6b, the third movable part 6c, the fourth movable part 6d, the fifth movable part 6e, and the sixth movable part 6f as the movable part 6, and the seventh movable part 9g, from the first detection unit 8a, the second detection unit 8b, the third detection unit 8c, the fourth detection unit 8d, the fifth detection unit 8e, and the sixth detection unit 8f, and the seventh detection unit 11g.

The master control unit 3 converts these detection signals into values in terms of amounts of movements of the movable parts of the slave device 2A and transmits the values to the slave device 2A.

To move the first movable part 6a, the second movable part 6b, the third movable part 6c, and the seventh movable part 9g, the master control unit 3 supplies control signals to the first drive unit 7a, the second drive unit 7b, the third drive unit 7c, and the seventh drive unit 10g, respectively.

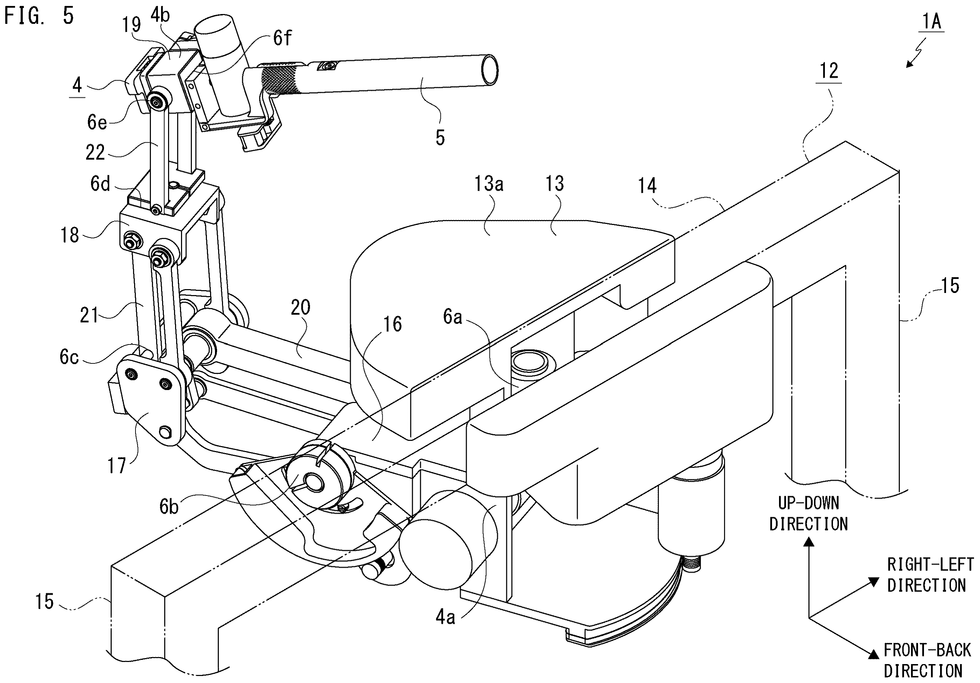

FIG. 5 is a perspective view of the master device 1A.

The master device 1A includes the master control unit 3, which is not illustrated in FIG. 5, and the arm unit 4 and the manipulation unit 5, which are illustrated in FIG. 5, and includes a frame part 12 that supports the arm unit 4, and an arm rest 13 that is attached to the frame part 12.

The frame part 12 indirectly supports the arm unit 4 with the arm rest 13 that is attached to the frame part 12 and to which the arm unit 4 is attached. The support of the arm unit 4 by the frame part 12 is not limited to this. A direct support of the arm unit 4 may be provided by attaching the arm unit 4 to the frame part 12.

Note that, in the following description, an up-down direction is defined such that a downward direction is a direction in which the arm unit 4 is attached to the frame part 12.

The frame part 12 includes an attachment portion 14 that extends horizontally and a pair of leg portions 15 that extends downward from both end portions of the attachment portion 14.

In the following description, a direction in which the attachment portion 14 of the frame part 12 extends is defined as a right-left direction, and a direction that is perpendicular to both the up-down direction and the right-left direction is defined as a front-back direction.

The arm rest 13 has an upper surface that serves as a placement surface 13a.

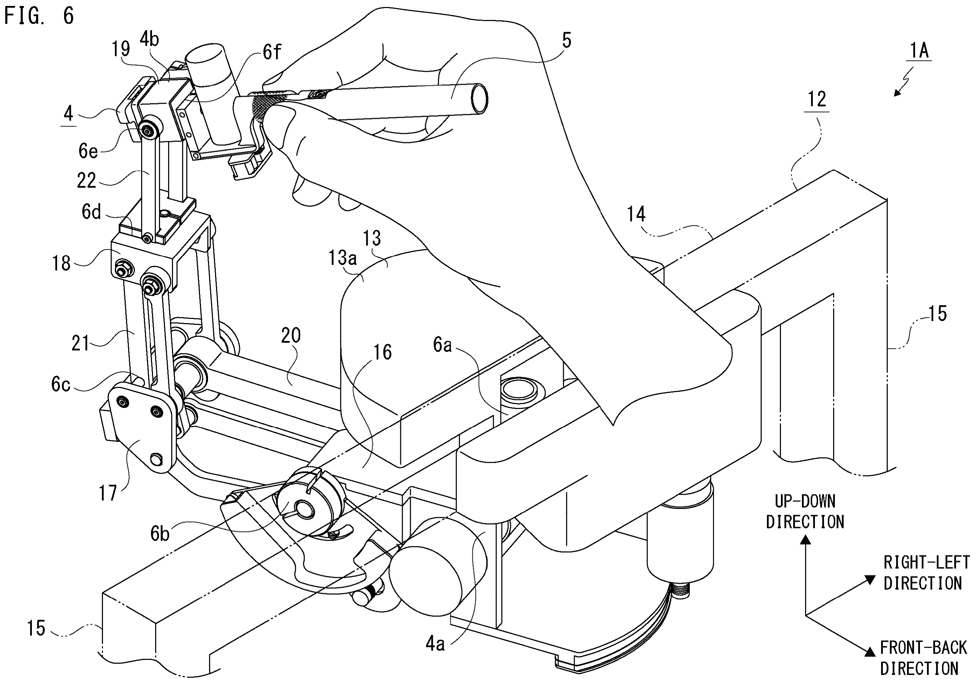

As illustrated in FIG. 6, the surgeon places a right wrist portion of the surgeon on the placement surface 13a of the arm rest 13 and grasps the manipulation unit 5 formed in a rod shape, with the body of the surgeon located backward of the frame part 12.

The arm unit 4 and the manipulation unit 5 move together in the right-left direction with respect to the attachment portion 14 of the frame part 12. The arm rest 13 is attached to the frame part 12 so as to move in the right-left direction with the movement of the arm unit 4 and the manipulation unit 5 in the right-left direction. That is, the arm unit 4, the manipulation unit 5, and the arm rest 13 slide together in the right-left direction with respect to the attachment portion 14.

Before performing surgery, the surgeon moves the arm unit 4, the manipulation unit 5, and the arm rest 13 to their suitable positions in the right-left direction and then fixes, with fixing means not illustrated, the positions of the arm unit 4, the manipulation unit 5, and the arm rest 13 in the right-left direction with respect to the attachment portion 14.

It is thus possible to prevent the arm unit 4, the manipulation unit 5, and the arm rest 13 from unexpectedly sliding in the right-left direction with respect to the attachment portion 14 during the surgery, thus preventing a manipulation mistake.

### <2-1. Configuration Example of Arm Unit>

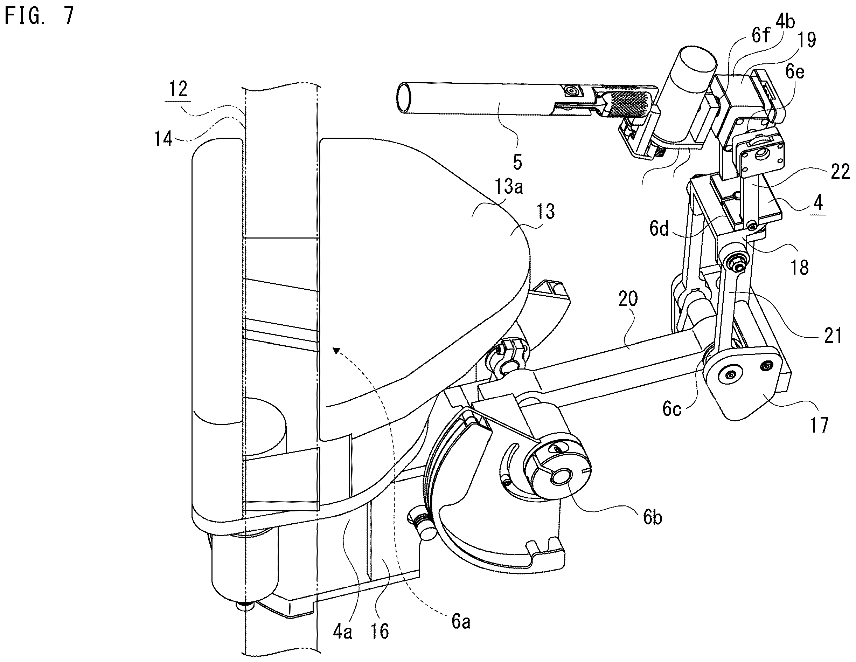

A specific configuration of the arm unit 4 will be described with reference to attached drawings. FIG. 7 is a perspective view of the arm unit 4 as viewed from the side in the right-left direction opposite to the side from which the arm unit 4 is viewed in FIG. 5.

As illustrated in FIG. 5 and FIG. 7, the movable parts 6 of the arm unit 4 are the first movable part 6a, the second movable part 6b, the third movable part 6c, the fourth movable part 6d, the fifth movable part 6e, and the sixth movable part 6f in this order from a root part 4a attached to the arm rest 13 to a terminal part 4b. That is, the first movable part 6a is the movable part 6 that is the closest to the root part 4a in the arm unit 4.

Specifically, the arm unit 4 includes a foundation 16 that is a part attached to the arm rest 13 and constitutes the root part 4a, a first intermediate part 17 that is provided closer to the terminal part 4b than the foundation 16, a second intermediate part 18 that is provided closer to the terminal part 4b than the first intermediate part 17, a support part 19 that constitutes the terminal part 4b and supports the manipulation unit 5, a first connection part 20 that connects the foundation 16 and the first intermediate part 17, a second connection part 21 that connects the first intermediate part 17 and the second intermediate part 18, and a third connection part 22 that connects the second intermediate part 18 and the support part 19.

The first movable part 6a of the arm unit 4 is provided as a part that turns with respect to the arm rest 13.

The second movable part 6b is provided as a part that turns the first connection part 20 with respect to the foundation 16.

The third movable part 6c is provided as a part that turns the second connection part 21 with respect to the first intermediate part 17.

The fourth movable part 6d is provided as a part that turns the third connection part 22 with respect to the second intermediate part 18.

The fifth movable part 6e is provided as a part that is turned to change the positional relationship between the second intermediate part 18 and the support part 19 in the third connection part 22.

The sixth movable part 6f is provided as a part that turns the manipulation unit 5 with respect to the support part 19.

### <2-1-1. First Movable Part>

First, the configuration of the first movable part 6a will be specifically described.

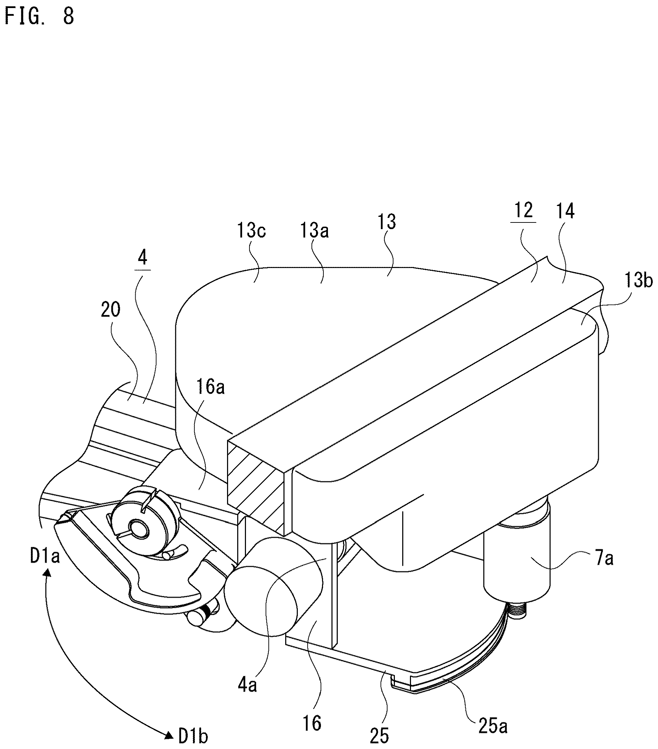

FIG. 8 is a perspective view illustrating part of the attachment portion 14 in the frame part 12, the arm rest 13, and the root part 4a and its vicinity in the arm unit 4. Note that the illustration of wiring and the like provided to move the first movable part 6a is omitted in FIG. 8. In the subsequent drawings, the illustration of wiring and the like provided to move the movable parts 6 are also omitted.

The arm rest 13 includes a near-side part 13b attached to the attachment portion 14 on the near side of the attachment portion 14 in the front-back direction, that is, the surgeon's side, and includes a far-side part 13c attached to the attachment portion 14 on the far side of the attachment portion 14 in the front-back direction. The placement surface 13a includes upper surfaces of the near-side part 13b and far-side part 13c.

The placement surface 13a is also a surface on which part of a forearm of the surgeon is placed and the part of the forearm slides when the forearm is moved. It is thus desirable that the placement surface 13a have low friction.

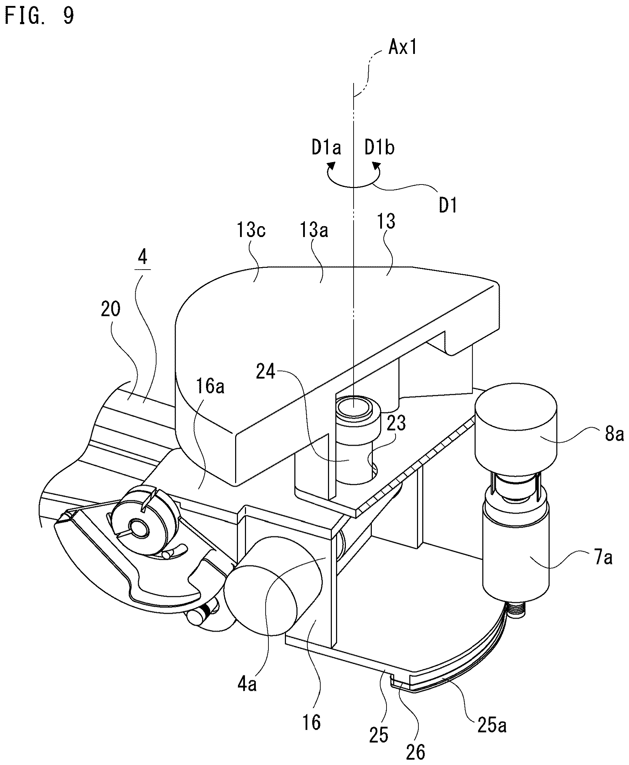

FIG. 9 is a perspective view illustrating the far-side part 13c of the arm rest 13, and the root part 4a and its vicinity in the arm unit 4. That is, FIG. 9 is a drawing in which the attachment portion 14 and the near-side part 13b in FIG. 8 are not illustrated.

As illustrated in FIG. 9, the far-side part 13c of the arm rest 13 is provided with an insertion opening 23 that is opened downward and formed in a cylindrical hole.

The foundation 16 of the arm unit 4 includes a base part 16a formed in a substantially box shape and includes a projecting portion 24 that is tubular and projects upward from the base part 16a. The projecting portion 24 is formed in a such a shape that makes the projecting portion 24 being inserted in the insertion opening 23 prevent the base part 16 from falling out downward through the insertion opening 23.

With the projecting portion 24 inserted in the insertion opening 23, the arm unit 4 is supported by the arm rest 13.

Being inserted in the insertion opening 23, the projecting portion 24 is turnable in directions D1 around a first turning axis Ax1, which is a common axis of the insertion opening 23 and the projecting portion 24.

That is, as illustrated in FIG. 8, FIG. 9, and FIG. 10, as the projecting portion 24 being inserted in the insertion opening 23 is turned in the direction around the first turning axis Ax1, the entire arm unit 4 is turned in the direction around the first turning axis Ax1 with respect to the arm rest 13. That is, the projecting portion 24 of the foundation 16 is provided as the first movable part 6a that moves with respect to the arm rest 13.

Note that there are various possible configurations of the insertion opening 23 and the projecting portion 24 for enabling the entire arm unit 4 to turn in the direction around the first turning axis Ax1 with respect to the arm rest 13 and preventing the foundation 16 from falling out of the arm rest 13, and the illustrated example is merely an example.

As described above, the arm unit 4 includes the first drive unit 7a as a mechanism that causes the arm unit 4 to turn in the directions D1 around the first turning axis Ax1. Note that, as viewed from above, the directions D1 around the first turning axis Ax1 include a clockwise direction denoted as D1a and a counterclockwise direction denoted as D1b.

As illustrated in FIG. 8 and FIG. 9, the first drive unit 7a is provided projecting downward from the near-side part 13b of the arm rest 13. For example, the first drive unit 7a is a motor with a cylindrical drive shaft of which an axial direction is the up-down direction.

The foundation 16 is provided with a portion that is to be driven by the first drive unit 7a. Specifically, on a lower end portion of the foundation 16, a laterally projecting portion 25 that projects in the opposite direction to a direction in which the first connection part 20 extends is formed. Note that, in the following description, for the base part 16a of the foundation 16, a direction in which the first connection part 20 extends will be denoted as a depth direction, and a horizontal direction that is perpendicular to the depth direction will be denoted as a width direction. That is, the depth direction and the width direction are directions that change in accordance with a state into which the foundation 16 turns with respect to the arm rest 13.

The laterally projecting portion 25 is provided projecting from the base part 16a in the depth direction.

The laterally projecting portion 25 includes an outer circumference surface 25a that is a surface forming an arc centered at the first turning axis Ax1 and is a surface facing a lateral side.

On the outer circumference surface 25a, a wire 26 are stretched along the arc. The wire 26 has one end that is attached to an end portion of the arc of the outer circumference surface 25a and the other end that is attached to the drive shaft of the motor as the first drive unit 7a.

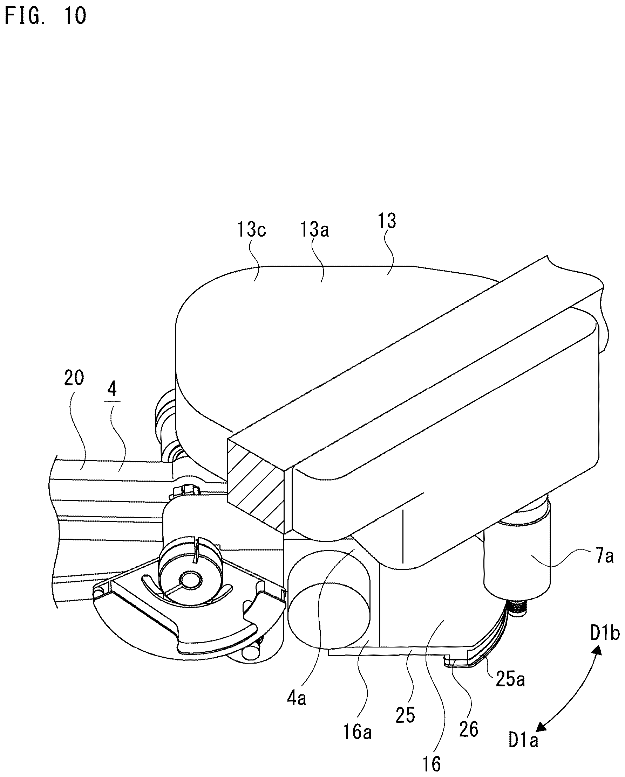

When the motor as the first drive unit 7a rotates in a predetermined direction, the wire 26 is wound around the shaft of the motor. This causes the foundation 16 to turn in the direction D1b around the first turning axis Ax1 as illustrated in FIG. 8 and FIG. 10.

Note that the foundation 16 is biased by a biasing member not illustrated, such as a spring, such that the foundation 16 turns in the direction D1a of the directions D1 around the first turning axis Ax1 with respect to the arm rest 13. As a result, when the motor as the first drive unit 7a rotates in the opposite direction to the predetermined direction, the wire 26 is unwound in accordance with the amount of the rotation, and the base part 16 is turned in the direction D1a, which is a direction of the biasing, by the biasing member such as a spring.

The amount of the turn of the foundation 16 with respect to the arm rest 13 can be detected by the first detection unit 8a as described above. As illustrated in FIG. 9, the first detection unit 8a is provided in a cylindrical shape, above and coaxially with the first drive unit 7a. The first detection unit 8a is capable of detecting the amount of driving or the amount of the rotation of the first drive unit 7a such as a motor. Note that the first detection unit 8a may be configured to be capable of detecting the angle of the foundation 16 with respect to the arm rest 13 so as to be capable of detecting the amount of the turn of the foundation 16 with respect to the arm rest 13.

### <2-1-2. Second Movable Part>

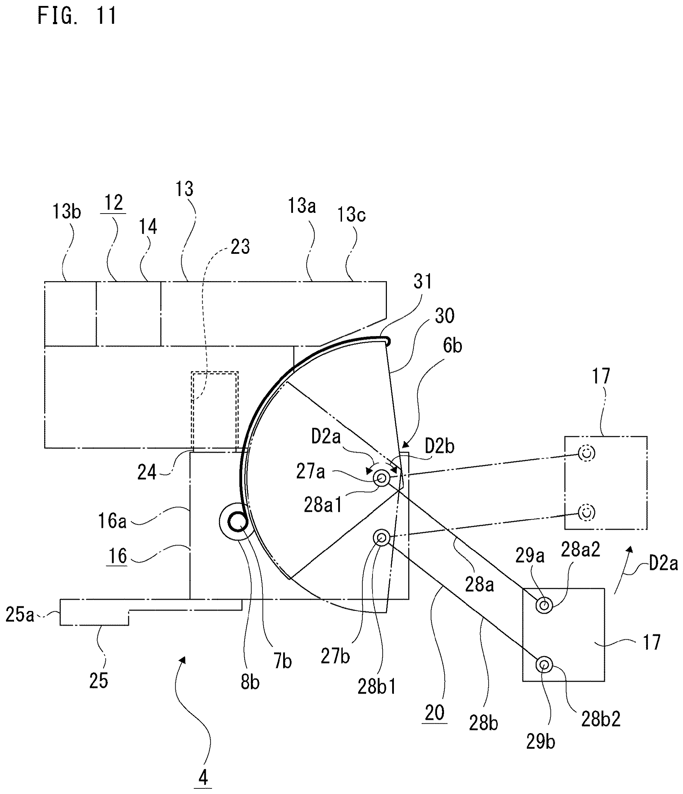

Next, the second movable part 6b will be described with reference to FIG. 11. Note that the second movable part 6b is a connection part between the foundation 16 and the first connection part 20. Note that the following description will be given with reference to drawings that are simpler than the above-described drawings as in FIG. 11.

FIG. 11 is a diagram illustrating the second movable part 6b as viewed from the opposite direction in the right-left direction to the direction from which the second movable part 6b is viewed in FIG. 8 and FIG. 9.

In FIG. 11, the illustration of the second intermediate part 18, the support part 19, and the second connection part 21 in the arm unit 4 and the illustration of the first drive unit 7a, the first detection unit 8a, and the like are omitted.

The foundation 16 is provided with two shaft parts 27, 27 that are provided closer to the terminal part 4b than the projecting portion 24 and each of which is formed in a rod shape.

The two shaft parts 27 are located separate from each other in the up-down direction, with their axial directions are the width direction of the base part 16a. The two shaft parts 27 are a shaft part 27a located at an upper position and a shaft part 27b located at a lower position.

The first connection part 20 includes two parallel links 28, 28. The two parallel links 28 are a parallel link 28a located at an upper position and a parallel link 28b located at a lower position.

To the shaft part 27a, which is located at an upper position of the two shaft parts 27, one end portion 28a1 of the parallel link 28a is attached.

To the shaft part 27b, which is located at a lower position of the two shaft parts 27, one end portion 28b1 of the parallel link 28b is attached.

The parallel link 28a is turnable in a direction around the shaft part 27a with respect to the shaft part 27a. That is, the parallel link 28a is turnable in the direction around the shaft part 27a with respect to the base part 16a of the foundation 16.

Likewise, the parallel link 28b is turnable in a direction around the shaft part 27b with respect to the shaft part 27b, thus being turnable around the shaft part 27b with respect to the base part 16a of the foundation 16.

The first intermediate part 17 is provided with two shaft parts 29, 29 each of which is formed in a rod shape. The axial directions of the shaft parts 29 match the axial directions of the shaft parts 27, that is, the width direction of the base part 16a.

To a shaft part 29a, which is located at an upper position of the two shaft parts 29, another end portion 28a2 of the parallel link 28a is attached.

To a shaft part 29b, which is located at a lower position of the two shaft parts 29, another end portion 28b2 of the parallel link 28b is attached.

The parallel link 28a is turnable in a direction around the shaft part 29a. That is, the parallel link 28a is turnable around the shaft part 29a with respect to the first intermediate part 17.

Likewise, the parallel link 28b is turnable in a direction around the shaft part 29b, thus being turnable around the shaft part 29b with respect to the first intermediate part 17.

The foundation 16 and the first intermediate part 17 being coupled together with the two parallel links 28a, 28b make only the position of the first intermediate part 17 with respect to the foundation 16 adjustable while the attitude of the first intermediate part 17 with respect to the foundation 16 is maintained.

The first intermediate part 17 turns with respect to the foundation 16, with the center of turn lying roughly in the vicinity of the midpoint between the shaft part 27a and the shaft part 27b. That is, the shaft parts 27, and the one end portion 28a1 of the parallel link 28a and the one end portion 28b1 of the parallel link 28b are provided as the second movable part 6b.

Note that, in FIG. 11, solid lines illustrate a state before the first intermediate part 17 turns with respect to the foundation 16, and dot-dot-dash lines illustrate a state after the turn.

A configuration of the second drive unit 7b for achieving the turning motion of the second movable part 6b will be described.

To the one end portion 28a1 of the parallel link 28a attached to the shaft part 27a, a turning member 30, of which a surface facing in the width direction of the base part 16a is formed as a surface in a sector shape, is attached. For example, the turning member 30 is attached coaxially with the shaft part 27a and turns together with the one end portion 28a1 in the direction around the shaft part 27a.

On a lateral surface forming the arc of the turning member 30, a wire 31 is stretched. One end of the wire 31 is fixed in the vicinity of one end of the arc of the turning member 30.

The other end of the wire 31 is attached to a drive shaft of the second drive unit 7b, such as a motor, arranged on the outer circumferential side of the turning member 30. In FIG. 11, the wire 31 is illustrated with a thick solid line.

When the motor as the second drive unit 7b rotates in a predetermined direction, the wire 31 is wound around the shaft of the motor. This causes the parallel link 28a to turn in a predetermined direction D2a with respect to the base part 16a.

Note that the parallel link 28a is biased by a biasing member not illustrated, such as a spring, such that the parallel link 28a turns with respect to the base part 16a in a direction D2b, which is the opposite direction to the direction D2a. As a result, when the motor as the second drive unit 7b rotates in the opposite direction to the predetermined direction, the wire 31 is unwound in accordance with the amount of the rotation, and the parallel link 28a is turned in the direction D2b, which is a direction of the biasing, by the biasing member such as a spring.

The parallel link 28b is turned with the turn of the parallel link 28a with respect to the foundation 16, thus being kept in parallel to the parallel link 28a.

The amount of the turn of the parallel link 28a with respect to the base part 16a of the foundation 16 can be detected by the second detection unit 8b. As illustrated in FIG. 11, the second detection unit 8b is provided coaxially with the second drive unit 7b. The second detection unit 8b is capable of detecting the amount of driving or the amount of the rotation of the second drive unit 7b such as a motor. Note that the second detection unit 8b may be configured to be capable of detecting the angle of the parallel link 28a with respect to the base part 16a.

### <2-1-3. Third Movable Part>

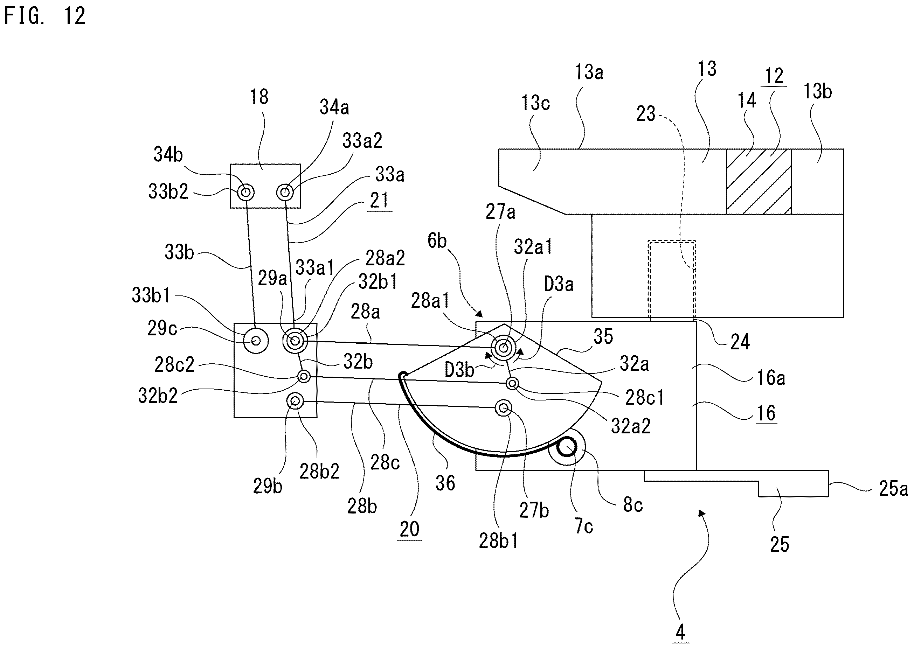

Next, the third movable part 6c will be described with reference to FIG. 12. The third movable part 6c is a connection part between the first intermediate part 17 and the second connection part 21.

FIG. 12 illustrates a configuration relating to the third movable part 6c, which is the configuration omitted in FIG. 11.

To the shaft part 27a attached to the base part 16a and extending in the width direction of the base part 16a, one end portion 32a1 of a parallel link 32a is further attached.

To the shaft part 29a attached to the first intermediate part 17, one end portion 32b1 of a parallel link 32b, which is parallel to the parallel link 32a, is further attached.

At another end portion 32a2 of the parallel link 32a and another end portion 32b2 of the parallel link 32b, shaft-shape portions projecting in the width direction of the base part 16a are formed.

To the shaft-shape portion formed at the other end portion 32a2 of the parallel link 32a and the shaft-shape portion formed at the other end portion 32b2 of the parallel link 32b, one end portion 28c1 and another end portion 28c2 of a parallel link 28c, which is parallel to the parallel link 28a, are attached.

That is, when viewed from a lateral side, the parallel link 28a, the parallel link 28c, the parallel link 32a, and the parallel link 32b form the sides of a parallelogram.

The one end portion 28c1 of the parallel link 28c is turnable with respect to the shaft-shape portion formed at the other end portion 32a2 of the parallel link 32a in the circumferential direction of the shaft-shape portion. That is, the angle formed between the parallel link 28c and the parallel link 32a as viewed from a lateral side is made adjustable.

The other end portion 28c2 of the parallel link 28c is turnable with respect to the shaft-shape portion formed at the other end portion 32b2 of the parallel link 32b in the circumferential direction of the shaft-shape portion.

The first intermediate part 17 is provided with a shaft part 29c that is formed in a rod shape, in addition to the shaft part 29a and the shaft part 29b. The axial direction of the shaft part 29c is the same as that of the shaft part 29a and the shaft part 29b and is the width direction of the base part 16a.

The second connection part 21 connecting the first intermediate part 17 and the second intermediate part 18 includes two parallel links 33. The two parallel links 33 are a parallel link 33a located relatively close to the foundation 16 and a parallel link 33b located relatively far from the foundation 16.

One end portion 33a1 of the parallel link 33a is connected to the one end portion 32b1 of the parallel link 32b. In the example illustrated in FIG. 12, the parallel link 32b and the parallel link 33a are located on the opposite sides of the shaft part 29a in the circumferential direction of the shaft part 29a. The parallel link 32b and the parallel link 33a are turned together in a direction around the shaft part 29a.

One end portion 33b1 of the parallel link 33b is turnably attached to the shaft part 29c.

The second intermediate part 18 is provided with two rod-shaped shaft parts 34 that extend in the width direction of the base part 16a. The two shaft parts 34 are a shaft part 34a located relatively close to the foundation 16 and a shaft part 34b located relatively far from the foundation 16.

To the shaft part 34a, another end portion 33a2 of the parallel link 33a is turnably attached.

To the shaft part 34b, another end portion 33b2 of the parallel link 33b is turnably attached.

The movement of the third movable part 6c will be described.

The parallel link 32a is turnable in a direction around the shaft part 27a with respect to the shaft part 27a.

When the parallel link 32a turns in one direction in the direction around the shaft part 27a, the parallel link 28c is moved in a direction substantially toward the surgeon in conjunction with the turn, and further the parallel link 32b is turned in the direction around the shaft part 29a.

When the parallel link 32b is turned in the direction around the shaft part 29a, the parallel link 33a is also turned in the direction around the shaft part 29a.

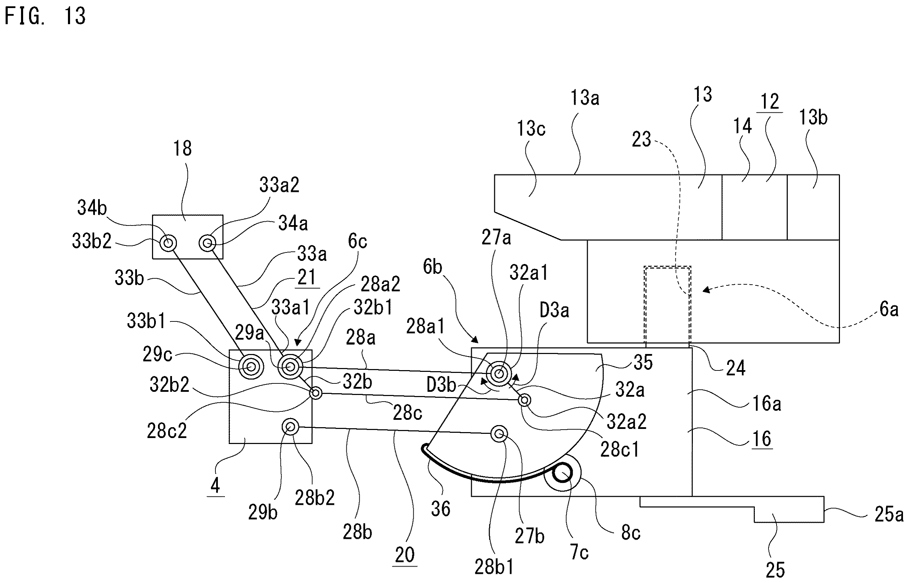

As illustrated in FIG. 13, this causes the second intermediate part 18 to turn with respect to the first intermediate part 17, with the center of turn lying roughly in the vicinity of the midpoint between the shaft part 29a and the shaft part 29c. Note that the first intermediate part 17 and the second intermediate part 18 being coupled together with the two parallel links 33a, 33b make only the position of the second intermediate part 18 with respect to the first intermediate part 17 adjustable while the attitude of the second intermediate part 18 with respect to the first intermediate part 17 is maintained.

As understood from the above, the shaft part 29a, the shaft part 29c, the one end portion 33a1 of the parallel link 33a, and the one end portion 33b1 of the parallel link 33b are provided as the third movable part 6c.

A configuration of the third drive unit 7c for achieving the turning motion of the third movable part 6c will be described.

To the one end portion 32a1 of the parallel link 32a attached to the shaft part 27a, a turning member 35, of which a surface facing in the width direction of the base part 16a is formed as a surface in a sector shape, is attached. For example, the turning member 35 is attached coaxially with the shaft part 27a and turns together with the one end portion 32a1 in the direction around the shaft part 27a.

Note that the turning member 35 is a member different from the turning member 30 in the description of the configuration of the second drive unit 7b.

For example, the turning member 30 is provided on one lateral surface of the base part 16a in the width direction, and the turning member 35 is provided on the other lateral surface.

In FIG. 12 and FIG. 13, the turning member 35 is illustrated while the turning member 30 is not illustrated.

On a lateral surface forming the arc of the turning member 35, a wire 36 is stretched. One end of the wire 36 is fixed in the vicinity of one end of the arc of the turning member 35.

The other end of the wire 36 is attached to a drive shaft of the third drive unit 7c, such as a motor, arranged on the outer circumferential side of the turning member 35.

When the motor as the third drive unit 7c rotates in a predetermined direction, the wire 36 is wound around the shaft of the motor. The turning member 35 turns in the direction around the shaft part 27a, and the one end portion 32a1 of the parallel link 32a turns in the direction around the shaft part 27a. This causes the parallel link 32a to turn with respect to the base part 16a in a predetermined direction D3a, switching from the state illustrated in FIG. 12 to the state illustrated in FIG. 13.

Note that the parallel link 32a is biased by a biasing member not illustrated, such as a spring, such that the parallel link 32a turns with respect to the base part 16a in a direction D3b, which is the opposite direction to the direction D3a. As a result, when the motor as the third drive unit 7c rotates in the opposite direction to the predetermined direction, the wire 36 is unwound in accordance with the amount of the rotation, and the parallel link 32a is turned in the direction D3b, which is a direction of the biasing, by the biasing member such as a spring.

The amount of the turn of the parallel link 32a with respect to the base part 16a of the foundation 16 can be detected by the third detection unit 8c. As illustrated in FIG. 12 and FIG. 13, the third detection unit 8c is provided coaxially with the third drive unit 7c. The third detection unit 8c is capable of detecting the amount of driving or the amount of the rotation of the third drive unit 7c such as a motor. Note that the third detection unit 8c may be configured to be capable of detecting the angle of the parallel link 32a with respect to the base part 16a.

Note that the third movable part 6c is one of the movable parts 6 that is provided together with the above-described first movable part 6a and second movable part 6b to principally make the position of the manipulation unit 5 adjustable.

Note that the positions of the shaft part 29a, the shaft part 29c, the one end portion 33a1 of the parallel link 33a, and the one end portion 33b1 of the parallel link 33b, which are provided as the third movable part 6c, are horizontally separate from a point at which the arm unit 4 is supported by the arm rest 13. Note that the point at which the arm unit 4 is supported by the arm rest 13 is the point at which the projecting portion 24 and the insertion opening 23 are connected.

On the other hand, the position of the third drive unit 7c such as a motor used to move the third movable part 6c is a position in proximity to the connection point of the projecting portion 24 and the insertion opening 23 in a horizontal direction.

The third drive unit 7c such as a motor is typically heavy. As a result, an increase in the horizontal distance between the third drive unit 7c and the connection point of the projecting portion 24 and the insertion opening 23 increases by leverage a force that tends to turn the first intermediate part 17 and the second intermediate part 18 downward, that is, a force that tends to turn the first intermediate part 17 in the direction D2b, thus making it difficult to keep the positions of the first intermediate part 17 and the parts arranged on the terminal side of the first intermediate part 17.

This also increases a driving force of the third drive unit 7c necessary to keep or assist in keeping the positions of the first intermediate part 17 and the parts arranged on the terminal side of the first intermediate part 17, resulting in an increase in the size of the third drive unit 7c.

To avoid such a vicious cycle, the third drive unit 7c is arranged at the position in proximity to the connection point of the projecting portion 24 and the insertion opening 23, in other words, at a position directly below the far-side part 13c of the arm rest 13.

This can reduce a force that tends to turn the first intermediate part 17 in the direction D2b due to gravity, and can further make it possible to reduce the third drive unit 7c in size.

### <2-1-4. Fourth Movable Part>

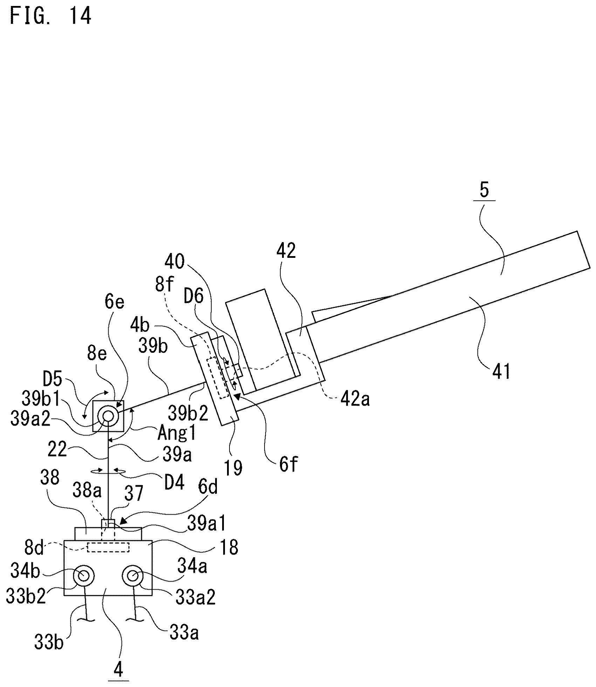

Next, the fourth movable part 6d will be described with reference to FIG. 14.

The fourth movable part 6d is a connection part between the second intermediate part 18 and the third connection part 22.

Note that, in FIG. 14, the illustration of the foundation 16, the first intermediate part 17, and the first connection part 20 is omitted.

On the second intermediate part 18, an engagement protrusion 37 that is in a cylindrical shape and projects upward is formed. Note that the term "upward" here means a direction in which the engagement protrusion 37 projects when the arm unit 4 assumes the attitude illustrated in FIG. 14. Accordingly, the projecting direction of the engagement protrusion 37 is not limited to the upward direction, and some attitudes of the arm unit 4 make the projecting direction diagonally upward.

The third connection part 22 includes an attachment mount part 38 attached to the second intermediate part 18 and includes two links. The two links included in the third connection part 22, links 39, are a proximal-side link 39a that is formed in a rod shape and extends from the attachment mount part 38, and a distal-side link 39b that connects the proximal-side link 39a and the support part 19.

At a substantially central portion of the attachment mount part 38, a through hole 38a that penetrates the attachment mount part 38 in its thickness direction is formed. The attachment mount part 38 is attached to the second intermediate part 18, with the engagement protrusion 37 inserted in the through hole 38a.

The attachment mount part 38 is turnable with respect to the second intermediate part 18 in directions D4 around the axis of the engagement protrusion 37.

That is, the engagement protrusion 37 of the second intermediate part 18 and the attachment mount part 38 with the through hole 38a are provided as the fourth movable part 6d.

Inside the second intermediate part 18, the fourth detection unit 8d that is capable of detecting the angle or the amount of the turn of the attachment mount part 38 with respect to the second intermediate part 18 is provided.

Note that the location where the fourth detection unit 8d is provided is not limited to the inside of the second intermediate part 18. The fourth detection unit 8d may be provided at any location at which the angle or the amount of the turn of the attachment mount part 38 with respect to the second intermediate part 18 can be detected.

### <2-1-5. Fifth Movable Part>

The fifth movable part 6e is provided as a part that changes the positional relationship between the second intermediate part 18 and the support part 19 in the third connection part 22. This will be specifically described with reference to FIG. 14.

Specifically, one end portion 39a1 of the proximal-side link 39a is connected to the attachment mount part 38. At another end portion 39a2 of the proximal-side link 39a, a shaft-shape portion that is in a cylindrical shape and extends laterally is formed.

The distal-side link 39b includes one end portion 39b1 turnably attached to the shaft-shape portion formed at the other end portion 39a2 of the proximal-side link 39a and includes another end portion 39b2 attached to the support part 19.

The distal-side link 39b is turnable with respect to the proximal-side link 39a in directions D5 around the axis of the shaft-shape portion provided at the other end portion 39a2. That is, an angle Ang1 formed between the proximal-side link 39a and the distal-side link 39b is adjustable.

The other end portion 39a2 of the proximal-side link 39a and the one end portion 39b1 of the distal-side link 39b are provided as the fifth movable part 6e.

At a connection part between the proximal-side link 39a and the distal-side link 39b, the fifth detection unit 8e that is capable of detecting the angle or the amount of the turn of the distal-side link 39b with respect to the proximal-side link 39a is provided.

### <2-1-6. Sixth Movable Part>

The sixth movable part 6f is a connection part between the support part 19 and the manipulation unit 5. This will be specifically described with reference to FIG. 14.

On the support part 19, an attachment projection 40 that is in a cylindrical shape and extends in the opposite direction to the distal-side link 39b is formed.

The manipulation unit 5 includes a pen-shaped grasping part 41 and a coupling part 42 that couples the grasping part 41 and the support part 19.

In the coupling part 42, an insertion hole 42a in which the attachment projection 40 of the support part 19 is inserted.

The coupling part 42 is turnable with respect to the support part 19 in directions D6 around the axis of the attachment projecting part 40, with the attachment projection 40 of the support part 19 inserted in the insertion hole 42a. The grasping part 41 is turned together with the coupling part 42 with respect to the support part 19.

Note that the grasping part 41 of the manipulation unit 5 is arranged coaxially with the attachment projection 40. As a result, the grasping part 41 turns in a direction around the central axis of the grasping part 41.

Inside the support part 19, the sixth detection unit 8f that is capable of detecting the angle or the amount of the turn of the manipulation unit 5 with respect to the support part 19 is provided.

Note that the location where the sixth detection unit 8f is provided is not limited to the inside of the support part 19. The sixth detection unit 8f may be provided at any location at which the angle or the amount of the turn of the manipulation unit 5 with respect to the support part 19 can be detected.

Note that the sixth movable part 6f is provided together with the fourth movable part 6d and the fifth movable part 6e to principally make the attitude of the manipulation unit 5 adjustable.

### <2-1-7. Seventh Movable Part>

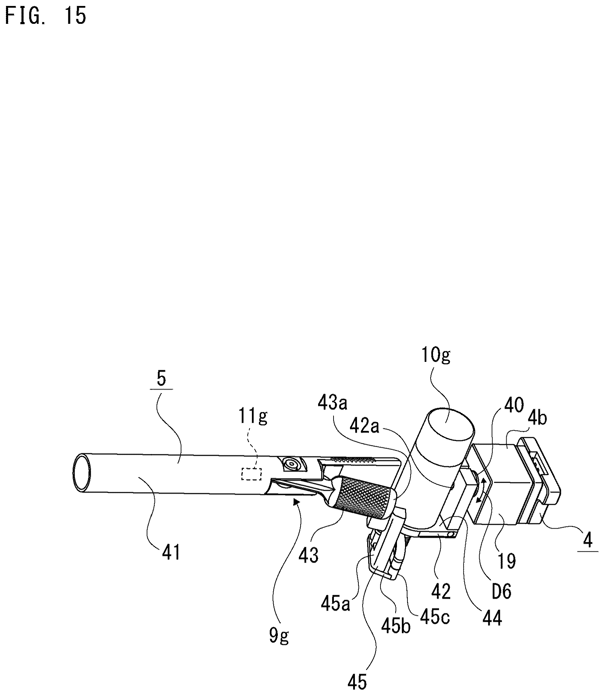

The seventh movable part 9g included in the manipulation unit 5 will be described with reference to FIG. 15, FIG. 16, FIG. 17, and the like. Note that FIG. 15, FIG. 16, and FIG. 17 are diagrams that illustrate only the manipulation unit 5 and the support part 19 out of the parts included in the master device 1A in an extractive manner.

On the grasping part 41 of the manipulation unit 5, a nail part 43 that projects outward from the vicinity of the central portion of the grasping part 41 is formed.

The nail part 43 is provided diagonally projecting from the grasping part 41 such that the nail part 43 becomes separated from the central axis of the grasping part 41 as the nail part 43 extends toward the support part 19.

As illustrated from FIG. 15 to FIG. 17, the amount of the separation of a tip portion 43a of the nail part 43 with respect to the grasping part 41 is adjustable. That is, the angle formed between the grasping part 41 and the nail part 43 is adjustable.

The connection part of the grasping part 41 to the nail part 43 is provided as the seventh movable part 9g.

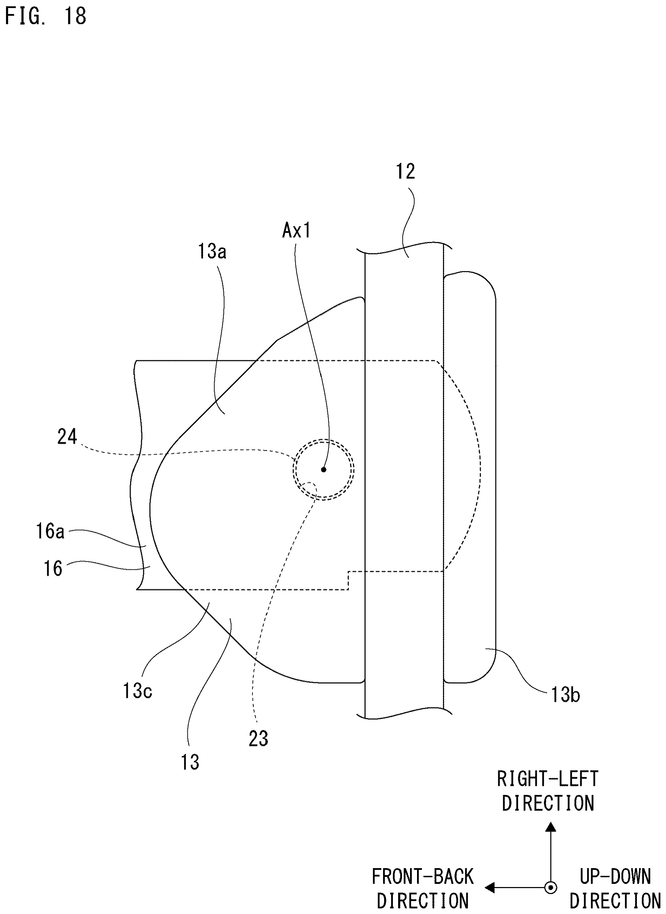

As illustrated in FIG. 18, the surgeon can perform such an operation that the nail part 43 is pushed with the surgeon's index finger or the like while the grasping part 41 is held with the surgeon's palm. In other words, an action of pinching an object with tweezers can be reproduced with the grasping part 41 and the nail part 43. In response to the action of pinching, an action of pinching an affected area or the like with the forceps as the surgical instrument held by the slave device 2A, an action of cutting an affected area with the scalpel as the held surgical instrument, or the like is implemented.

The manipulation unit 5 is provided with the drive unit 10g that drives the seventh movable part 9g for implementing the kinesthetic sense presentation function F2.

Specifically, in the coupling part 42, an arrangement recess 44 that is opened in three of four directions that are perpendicular to the turning axis of the sixth movable part 6f is formed. On the arrangement recess 44, the seventh drive unit 10g, such as a motor, is attached.

In the arrangement recess 44, a shaft insertion hole 44a in which the drive shaft of the seventh drive unit 10g is inserted is formed. The shaft insertion hole 44a is formed as a hole that penetrates in a direction perpendicular to the turning axis of the sixth movable part 6f.

The manipulation unit 5 is provided with a transmission part 45 that transmits motive power from the drive shaft of the seventh drive unit 10g. The transmission part 45 includes a first surface portion 45a and a second surface portion 45b. The first surface portion 45a and the second surface portion 45b extend in directions perpendicular to each other, thus forming an L shape.

The first surface portion 45a has one end coupled to the tip portion of the nail part 43 and has the other end provided continuously from the second surface portion 45b.

The second surface portion 45b includes one end provided continuously from the second surface portion 45b and includes the other end including a driven surface 45c that forms an arc centered at the proximal end portion of the nail part 43.

The driven surface 45c is provided with part of the driven surface 45c facing the outer circumference surface of the drive shaft of the seventh drive unit 10g. On the driven surface 45c, a wire 46 is stretched along the arc of the driven surface 45c. The wire 46 has one end that is attached to an end portion of the arc of the driven surface 45c and the other end that is attached to the drive shaft of the motor as the seventh drive unit 10g.

By the drive of the seventh drive unit 10g or an operation by the surgeon, the transmission part 45 and the nail part 43 are turned together, with the center of turn lying at the proximal end portion of the nail part 43.

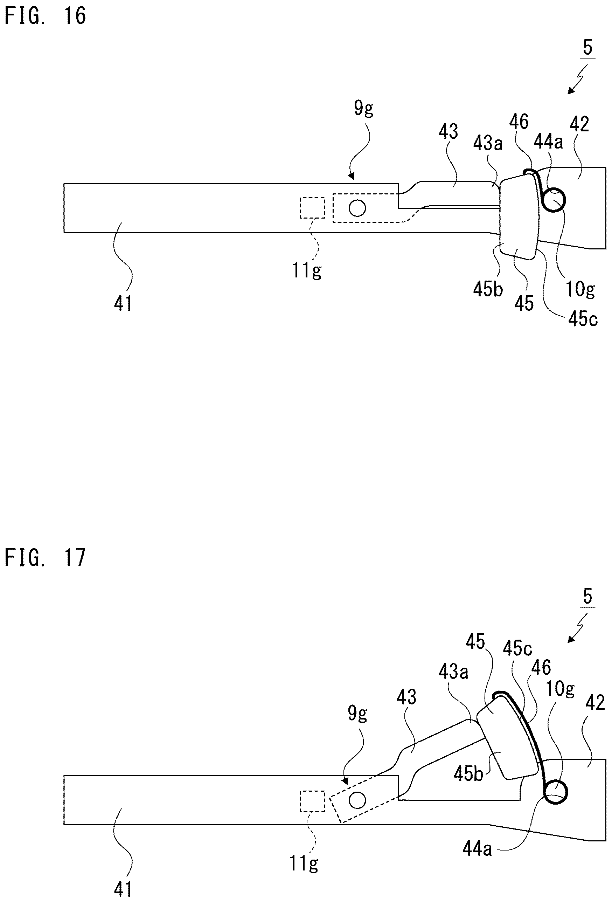

For example, when the motor as the seventh drive unit 10g rotates in a predetermined direction, the wire 26 is wound around the shaft of the motor. As illustrated in FIG. 16 to FIG. 17, this causes the transmission part 45 and the nail part 43 to turn together, with the center of turn lying at the proximal end portion of the nail part 43, and the nail part 43 shifts to the state in which the tip portion of the nail part 43 is separate from the grasping part 41.

When the surgeon performs an operation of pushing the nail part 43 with its tip portion separate from the grasping part 41 toward the grasping part 41, the nail part 43 shifts from the state in which the nail part 43 is separate from the grasping part 41 to the state in which the nail part 43 is close to the grasping part 41 as illustrated from FIG. 17 to FIG. 16.

At this time, in the master control unit 3, the seventh drive unit 10g exhibits the kinesthetic sense presentation function F2 by changing a driving force of the motor as the seventh drive unit 10g in accordance with a resistance received by the surgical instrument being manipulated, such as the forceps, which is held by the slave device 2A.

Inside the grasping part 41 of the manipulation unit 5, the seventh detection unit 11g that is capable of detecting the angle or the amount of the turn of the nail part 43 with respect to the grasping part 41 is provided. Note that the location where the seventh detection unit 11g is provided is not limited to the inside of the grasping part 41. The seventh detection unit 11g may be provided at any location at which the angle or the amount of the turn of the nail part 43 with respect to the grasping part 41 can be detected.

### <3. First Turning Axis>

The first turning axis Ax1, which is an axis relating to the movement of the first movable part 6a and is a turning axis of the foundation 16 turning with respect to the arm rest 13, will be described with reference to attached drawings.

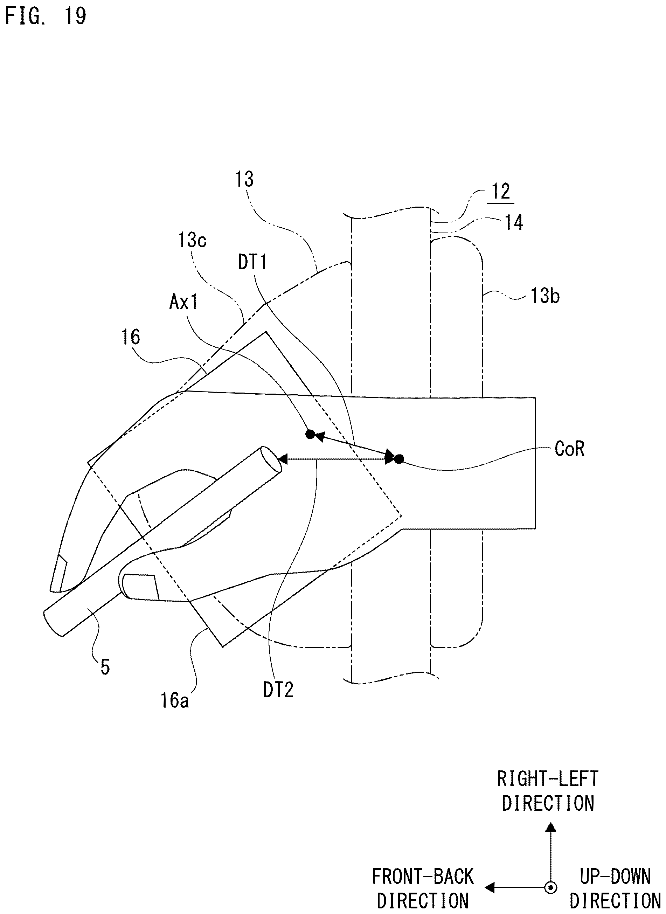

As illustrated in FIG. 9 and FIG. 19, the first turning axis Ax1 is an axis that extends in the up-down direction and passes through the arm rest 13. More specifically, the first turning axis Ax1 is an axis that passes through the far-side part 13c of the arm rest 13.

The first turning axis Ax1 can be considered as an axis that passes inside the contour of the arm rest 13 when viewed from above. That is, the first turning axis Ax1 may be an axis that passes through the attachment portion 14 of the frame part 12 provided between the near-side part 13b and the far-side part 13c.

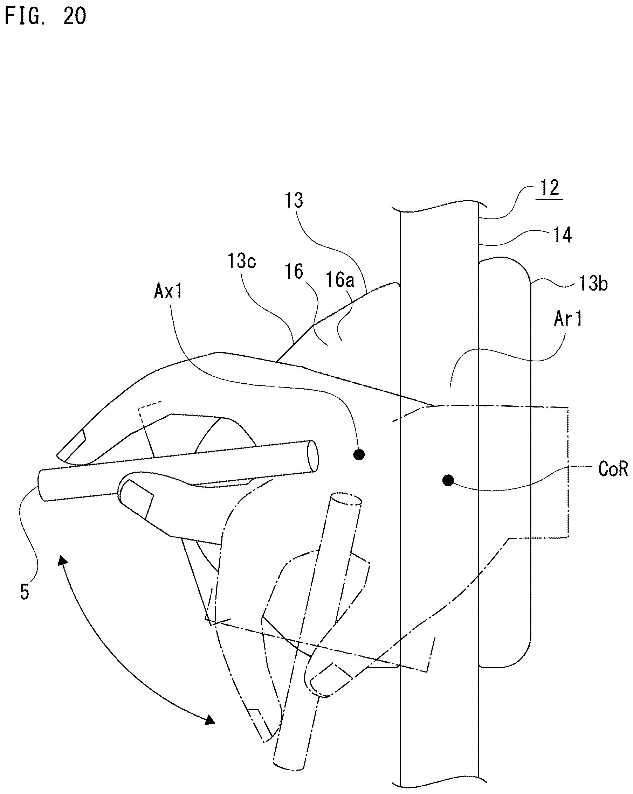

States before and after the foundation 16 turns with respect to the arm rest 13 are illustrated in FIG. 20. Note that manipulation of turning the foundation 16 with respect to the arm rest 13 will be denoted here as a first turning manipulation.

In FIG. 20, solid lines illustrate the state before the first turning manipulation and dot-dash lines illustrate the state after the first turning manipulation.

A center of turn CoR of the surgeon's arm in the first turning manipulation is, for example, in the vicinity of a wrist.

As illustrated in FIG. 19, the distance between the first turning axis Ax1 and the center of turn CoR is shorter than the distance between the manipulation unit 5 and the center of turn CoR. Specifically, assuming that a distance DT1 denotes the distance between the first turning axis Ax1 and the center of turn CoR, and that a distance DT2 denotes the distance between the center of turn CoR and the manipulation unit 5, the distance DT1 is shorter than the distance DT2.

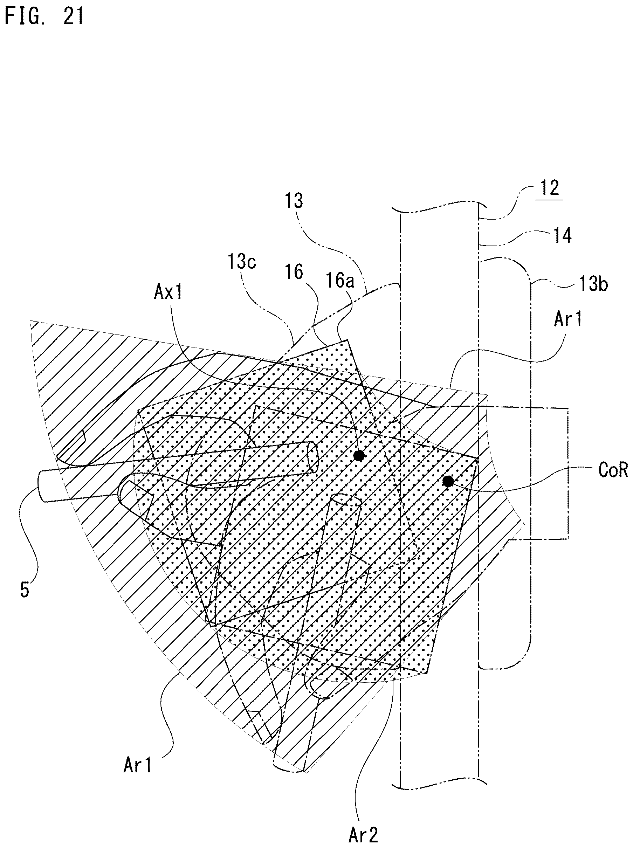

This makes most of a region Ar1 where a portion distal to a wrist of the surgeon can be present in the first turning manipulation and most of a region Ar2 where the base part 16a of the foundation 16 can be present overlap each other as viewed from above (see FIG. 21).

For example, in the case where the master device 1A is used to manipulate the slave device 2A that is remotely located, for performing surgery, various devices including a monitor displaying the states of the slave device 2A and a patient are installed close to the master device 1A.

Such various devices need to be installed at positions determined such that the devices are located in proximity to the master device 1A and do not obstruct the first turning manipulation. Specifically, the various devices need to be installed at positions at which the devices do not interfere with at least both the region Ar1 and the region Ar2.

Making most of the region Ar1 and most of the region Ar2 overlap each other enables a reduction in the size of a place where the master device 1A is installed. That is, this expands the range of regions where the various devices and the like can be installed, that is, the range of positions that overlap neither the region Ar1 nor the region Ar2 increase, and it is thus possible to provide a suitable manipulation environment.

In other words, it can be considered that the position of the first turning axis Ax1 is set such that the distance between the first turning axis Ax1 and the center of turn CoR is shorter than the distance between a grasped portion P1 in the grasping part 41 of the manipulation unit 5 and the center of turn CoR.

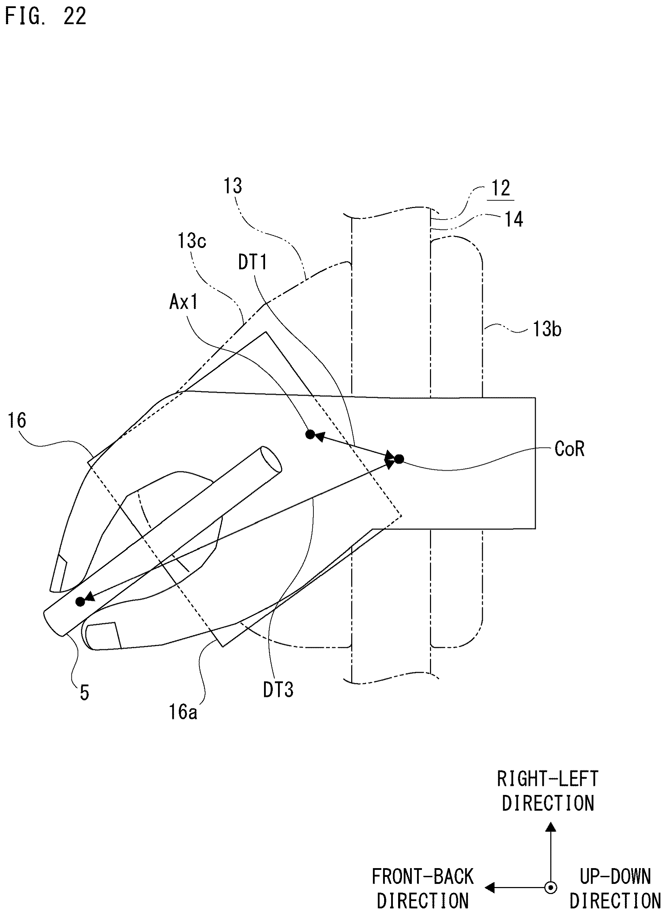

Specifically, as illustrated in FIG. 22, assuming that a distance DT3 denotes the distance between the grasped portion P1 of the grasping part 41, which is grasped with the surgeon's thumb and index finger, and the center of turn CoR, the distance DT1 is shorter than the distance DT3.

### <4. Modification Example>

In the above-described example, the master device 1A that is not provided with drive units 7 corresponding to the fourth movable part 6d, the fifth movable part 6e, and the sixth movable part 6f has been described.

The master device 1A is not limited to this and may be provided with the respective drive units 7 corresponding to the fourth movable part 6d, the fifth movable part 6e, and the sixth movable part 6f. In this case, a tactile sensation and a pressure such as a resistance force that are received by the surgical instrument of the slave device 2A are fed back to the surgeon by driving not only the first movable part 6a, the second movable part 6b, and the third movable part 6c but also the fourth movable part 6d, the fifth movable part 6e, and the sixth movable part 6f.

In the above-described example, the arm rest 13 is divided into the near-side part 13b and the far-side part 13c, and the placement surface 13a includes the upper surfaces of the near-side part 13b and the far-side part 13c. In addition, the upper surface of the attachment portion 14 of the frame part 12 is also a portion on which the surgeon's hand and the like is placed.

In this case, the surgeon may feel a difference in level between the near-side part 13b and the attachment portion 14 or a difference in level between the far-side part 13c and the attachment portion 14, and the feeling may affect the manipulation.

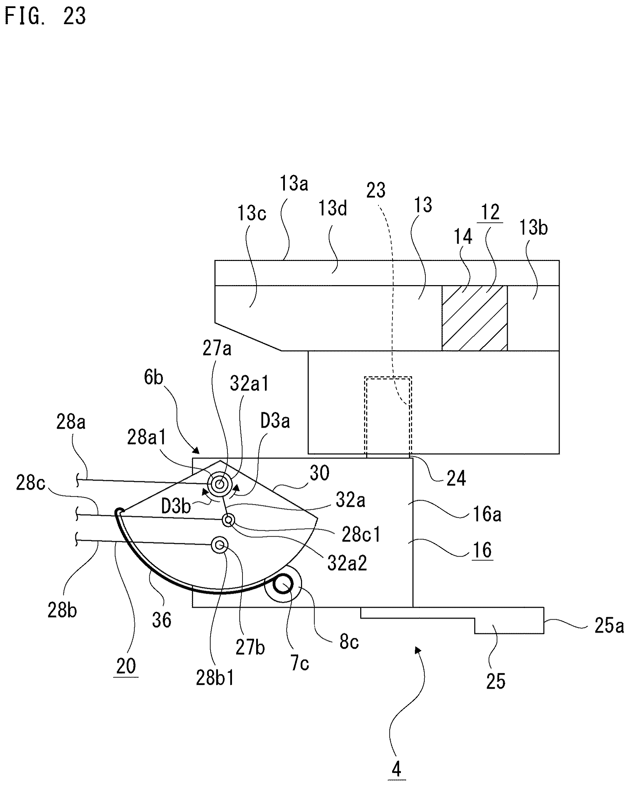

In the present modification example illustrated in FIG. 23, the arm rest 13 includes, in addition to the near-side part 13b and the far-side part 13c, an upper surface part 13d that is arranged so as to cover the near-side part 13b, the far-side part 13c, and a portion of the frame part 12 from above. The upper surface of the upper surface part 13d serves as the placement surface 13a.

This enables the surgeon to smoothly move the surgeon's hand or the like placed on the placement surface 13a, without feeling differences in level among the near-side part 13b, the attachment portion 14, and the far-side part 13c.

Note that the upper surface part 13d may be separately formed from the near-side part 13b and the far-side part 13c and attached to the near-side part 13b and the far-side part 13c from above.

Alternatively, the upper surface part 13d may be formed together with the near-side part 13b and the far-side part 13c.

The movable parts 6 and the movable part 9 provided at parts of the master device 1A described above may be restricted in their movements. Specifically, each of the movable parts 6 and the movable part 9 may be provided with a restricting mechanism so as to be capable of turning only within a predetermined range of angle.

For example, in the case of the first movable part 6a, a projection for restriction protruding upward is provided in the base part 16a of the foundation 16, and a restricting recess formed in a circular-arc-shaped groove is provided at a position corresponding to the projection for restriction in the far-side part 13c of the arm rest 13. By assembling the arm rest 13 and the foundation 16 such that the projection for restriction provided in the base part 16a and the restricting recess provided in the far-side part 13c are engaged with each other, it is possible to confine the range of turn of the foundation 16 with respect to the arm rest 13 in accordance with the length of the restricting recess being a groove. Similar mechanisms can be applied to the other movable parts 6 and the movable part 9, such as the second movable part 6b and the third movable part 6c.

Note that, as the restricting mechanism, a combination not including a projection and a recess, such as a configuration in which the range of turn is confined by means of the interference between plate-shaped protrusions, may be adopted.

Note that the surgery assisting system SA may be configured such that an amount of manipulation of the master device 1A by the surgeon is freely scaled up or down to an amount of movement in the slave device 2A.

For example, an amount of manipulation of one millimeter in the master device 1A may be scaled to an amount of movement of one micrometer of a corresponding movable part in the slave device 2A.

In the case where manipulation of the master device 1A is the manipulation of a ten-degree turn, a corresponding movable part of the slave device 2A may be turned by one degree.

### <5. Application Example>

The above-described example is the example in which the master device 1A is used by a surgeon such as a doctor to realize surgery with the slave device 2A.

The master device 1A is not limited to this and can be used as various devices.

For example, the manipulation assisting system S may include the slave device 2A that implements a task involving detailed work and the master device 1A that issues operation instructions to the slave device 2A.

Alternatively, the master device 1A and the slave device 2A may be used as a drawing device that draws a detailed picture. The detailed picture can be drawn with a pen held by the slave device 2A moving on the basis of manipulation on the master device 1A.

The provision of the master device 1A and the slave device 2A are not limited to the provision of them as a pair. For example, a controller device 1B as a modification example of the master device 1A may be provided as an input device for a computer device.

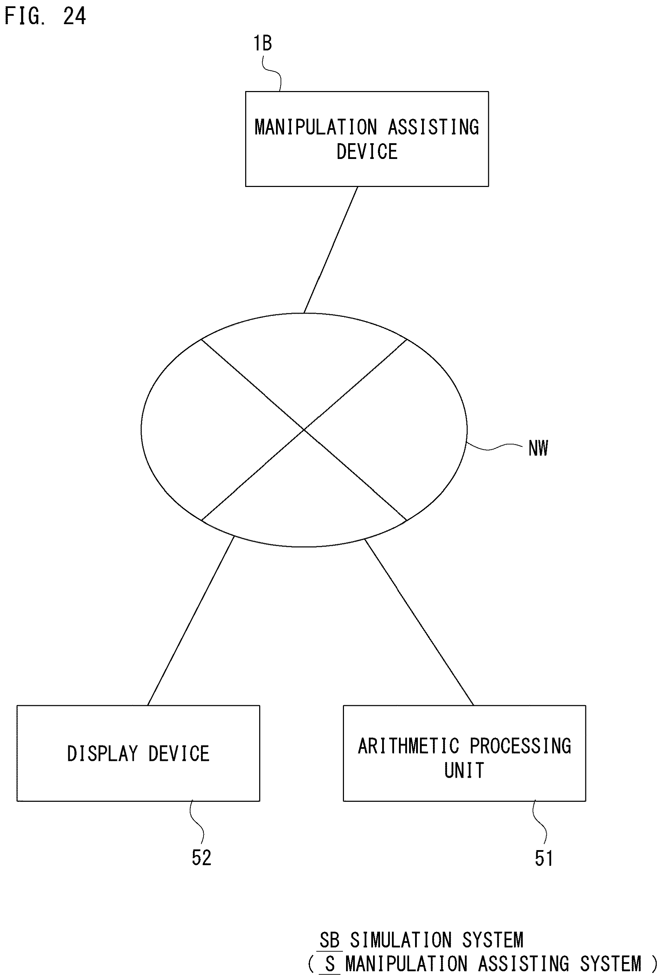

For example, as illustrated in FIG. 24, a simulation system SB, which is an aspect of the manipulation assisting system S, may include the controller device 1B that is provided as an instructing device in a virtual space that imitates an actual space, an arithmetic processing unit 51 that performs various computations on the basis of manipulations on the controller device 1B, and a display device 52 that displays the result of computation by the arithmetic processing unit 51.

The simulation system SB in such an aspect is, for example, a surgery simulation system used for improving techniques of surgery. That is, a manipulator who intends to improve their techniques of surgery manipulates the controller device 1B while recognizing a virtual patient displayed on the display device 52.

The arithmetic processing unit 51 computes the position and attitude of forceps, a scalpel, or the like in the virtual space on the basis of manipulations on the controller device 1B. The arithmetic processing unit 51 then generates an image to be displayed on the display device 52 on the basis of the result of computation.

The image generated by the arithmetic processing unit 51 is transmitted to the display device 52 and displayed on a display part. This enables the manipulator to visually check the image displayed on the display part of the display device 52, thus grasping the result of the manipulator's manipulation.

Alternatively, the simulation system SB may be a game system for achieving a predetermined goal in a virtual space. That is, a game user uses the controller device 1B as a controller for a manipulation object, such as a character, in the virtual space displayed on the display device 52.

On the basis of information on an input into the controller device 1B, the arithmetic processing unit 51 performs arithmetic processing and makes the virtual space to reflect the result of computation. The arithmetic processing unit 51 generates an image of the virtual space reflecting the result of computation and provides the image to the display device 52.

The display device 52 displays the image provided by the arithmetic processing unit 51 on the display part as the result of the input into the controller device 1B.

The game user can grasp the result of their manipulation by visually checking the image displayed on the display device 52.

### <6. Summary>

The manipulation assisting device as the master device 1A included in the surgery assisting system SA or the controller device 1B included in the simulation system SB described above includes the manipulation unit 5 to be manipulated by the manipulator (the surgeon or the game user), the arm unit 4 including the first movable part 6a turnable in the direction around the first turning axis Ax1 and making the position and attitude of the manipulation unit 5 adjustable, and the placement part (the arm rest 13) provided with the placement surface 13a on which part of a forearm used for manipulation of the manipulation unit 5 by the manipulator is to be placed. The first turning axis Ax1 is located at a position at which the distance between the first turning axis Ax1 and a wrist of a forearm of the manipulator is shorter than the distance between the manipulation unit 5 and the wrist when manipulation of the manipulation unit 5 is performed by a turn in which the wrist of the forearm is the center of turn CoR, with the forearm placed on the placement surface 13a.

Assuming that a "wrist turning manipulation" denotes a manipulation of the manipulation unit 5 by a turn in which the wrist is the center of turn, the above-described configuration makes a large part of the area Ar1 within which a hand moves in the wrist turning manipulation and a large part of an area within which the arm unit 4 moves in the wrist turning manipulation (e.g., the area Ar2 within which 16a moves) overlap with each other.

As a result, it is possible to reduce the ranges of movements of the manipulation assisting device and the manipulator, enabling the manipulation assisting device to be installed in a narrower place.

As described with reference to FIG. 19 and the like, in the manipulation assisting device as the master device 1A or the controller device 1B, the first turning axis Ax1 may be an axis that passes through the placement surface 13a.

This makes it possible to achieve a configuration that reduces the ranges of movements of the manipulation assisting device and the manipulator, enabling the manipulation assisting device to be installed in a narrower place.

As described with reference to FIG. 19 and the like, in the manipulation assisting device as the master device 1A or the controller device 1B, the first turning axis Ax1 may be an axis that is perpendicular to the placement surface 13a.

This makes the region Ar1 of the wrist and the region Ar2 of the base part 16a of the arm unit 4 extend horizontally.

In this case, setting the first turning axis Ax1 such that most of the region Ar1 and most of the region Ar2 overlap each other makes it possible to receive the full benefit of the effect of allowing places where the various devices installed around the manipulation assisting device to be increased in size.

As described with reference to FIG. 7 and the like, the first movable part 6a of the manipulation assisting device as the master device 1A or the controller device 1B may be installed on the side of the placement part (the arm rest 13) opposite to the placement surface 13a.

This enables the adoption of a configuration in which a structure is not provided immediately above the placement part. That is, the structure that can hinder the surgeon or the like from checking what the surgeon is doing need not be provided, which makes it easy for the manipulator to perform the manipulation.

As described with reference to FIG. 7, the placement part (the arm rest 13) in the manipulation assisting device as the master device 1A or the controller device 1B may be provided with a connection portion (the insertion opening 23) to which the arm unit 4 is connected, on the opposite side to the placement surface 13a, and the first movable part 6a may be provided at the closest position to the connection portion of the positions of the plurality of movable parts 6 included in the arm unit 4, that is, on the root part 4a side.

This facilitates an arrangement in which the first turning axis Ax1 is set at a position close to the center of turn CoR.

As described with reference to FIG. 1 and the like, the manipulation assisting device may be provided as the master device 1A that remotely manipulates the slave device 2A.

This makes it possible to reduce the size of the master device 1A for moving movable parts 9 of the slave device 2A that is remotely located.

As described with reference to FIG. 5 and the like, the manipulation assisting device as the master device 1A or the controller device 1B may include the frame part 12 to which the placement part (the arm rest 13) is attached, the position of the placement part with respect to the frame part 12 may be adjustable, and the arm unit 4 may be located at a position corresponding to the position of the placement part with respect to the frame part 12.

That is, the arm unit 4 and the manipulation unit 5 may be attached together to the attachment portion 14 of the frame part 12 in a slidable manner.

This makes it possible to move the arm unit 4 and the manipulation unit 5 to a position where the manipulator such as the surgeon easily performs the manipulation, enabling suitable assistance in a fine manipulation.

In particular, in the case where the right-hand arm unit 4R and the left-hand arm unit 4L are provided, it is possible to adjust, for example, the distance between the right-hand arm unit 4R and the left-hand arm unit 4L for each individual, thus making it possible to improve the ease of work.

Note that only one of the right-hand arm unit 4R or the left-hand arm unit 4L may be movably attached to the attachment portion 14.

As described with reference to FIG. 3 and the like, the manipulation assisting device as the master device 1A or the controller device 1B may be provided with the manipulation unit 5, the arm unit 4, the placement part (the arm rest 13) for the right hand, and with the manipulation unit 5, the arm unit 4, the placement part (the arm rest 13) for the left hand. That is, the surgery assisting system SA may be provided with the right-hand manipulation unit 5R, the right-hand arm unit 4R, and a right-hand arm rest 13, and the left-hand manipulation unit 5L, the left-hand arm unit 4L, and a left-hand arm rest 13.

This enables suitable assistance in surgery or work performed with both hands. Furthermore, a reduction in the ranges of movements of the arm unit 4 and the manipulation unit 5 for the right hand and the arm unit 4 and the manipulation unit 5 for the left hand makes an interference between right-hand parts and left-hand parts less likely to occur. As a result, it is easy to arrange the right-hand parts and the left-hand parts, and it is possible to prevent the entire device as the master device 1A or the controller device 1B from increasing in size.

As described with reference to FIG. 24 and the like, the manipulation assisting device may be provided as a controller (the controller device 1B) in the simulation system SB including the controller and the arithmetic processing unit 51 that performs a process of performing computation based on a manipulation on the controller and displaying the result of the computation on the display device 52.

This makes it possible to reduce the size of the manipulation assisting device (the controller device 1B) in the simulation system, lightening the restriction on the installation of the manipulation assisting device.

Note that the various examples described above can be freely combined.

### Reference Signs List

- S: manipulation assisting system
- SA: surgery assisting system
- SB: simulation system
- 1A: master device (manipulation assisting device)
- 1B: controller device (manipulation assisting device)
- 2A: slave device
- 4: arm unit
- 4L: left-hand arm unit (arm unit)
- 4R: right-hand arm unit (arm unit)
- 5: manipulation unit
- 5L: left-hand manipulation unit (manipulation unit)
- 5R: right-hand manipulation unit (manipulation unit)
- 6a: first movable part
- 12: frame part
- 13: arm rest (placement part)
- 13a: placement surface
- 23: insertion opening (connection portion)
- Ax1: first turning axis
- CoR: center of turn

## Claims

1. A manipulation assisting device comprising:
a manipulation unit to be manipulated by a manipulator;
an arm unit including a first movable part and making a position and an attitude of the manipulation unit adjustable, the first movable part being turnable in a direction around a first turning axis; and
a placement part including a placement surface on which part of a forearm used in the manipulation of the manipulation unit by the manipulator is to be placed, wherein
the first turning axis is located at a position at which a distance between the first turning axis and a wrist of a forearm of the manipulator is shorter than a distance between the manipulation unit and the wrist when a manipulation of the manipulation unit is performed by a turn in which the wrist is a center of turn, with the forearm placed on the placement surface.

2. The manipulation assisting device according to claim 1, wherein
the first turning axis is an axis that passes through the placement surface.

3. The manipulation assisting device according to claim 1, wherein
the first turning axis is an axis that is perpendicular to the placement surface.

4. The manipulation assisting device according to claim 1, wherein
the first movable part is provided on a side of the placement part opposite to the placement surface.

5. The manipulation assisting device according to claim 4, wherein
the placement part includes a connection portion to which the arm unit is connected, the connection portion being provided on the side opposite to the placement surface, and
the first movable part is provided at a position closest to the connection portion of positions of a plurality of movable parts included in the arm unit.

6. The manipulation assisting device according to claim 1, wherein
the manipulation assisting device is provided as a master device that remotely manipulates a slave device.

7. The manipulation assisting device according to claim 1, comprising
a frame part to which the placement part is attached, wherein
a position of the placement part with respect to the frame part is adjustable, and
the arm unit is located at a position corresponding to the position of the placement part with respect to the frame part.

8. The manipulation assisting device according to claim 1, wherein
the manipulation unit, the arm unit, and the placement part are provided for each of a right hand and a left hand.

9. The manipulation assisting device according to claim 1, wherein
the manipulation assisting device is provided as a controller in a simulation system including the controller and an arithmetic processing unit that performs a process of performing computation based on a manipulation on the controller and displaying a result of computation on a display device.
